# EUROPEAN PATENT APPLICATION

(11) **EP 4 732 751 A1**
(43) Date of publication of application: **29.04.2026**
(21) Application number: 24863281.2
(22) Date of filing: 06.09.2024
(51) Int. Cl.: A61B 1/06, A61B 1/00

(54) **ENDOSCOPE**

(30) Priority: 08.09.2023 KR 20230119400
(71) Applicant: LG Electronics Inc., Yeongdeungpo-gu Seoul 07336 (KR)
(72) Inventor: KIM, Wonsu, Seoul 06772 (KR); HWANG, Changkyu, Seoul 06772 (KR); KIM, Soonbeom, Seoul 06772 (KR)
(74) Representative: Vossius & Partner Patentanwälte Rechtsanwälte mbB
(86) International application number: PCT/KR2024/013558
(87) International publication number: WO 2025/053702

(57) **Abstract**

There is disclosed an endoscope, including: an insertion unit including an illumination and imaging unit and an insertion tube; and an operating unit provided with an operating knob and configured to perform a bending operation of the insertion tube according to an operation of the operating knob, wherein the illumination and imaging unit includes an illumination and imaging unit body, an illumination module configured to illuminate light, a heat sink in contact with the illumination module, and a heat dissipation chassis provided to surround an outer circumferential surface of the illumination and imaging unit body and disposed to contact the heat sink to dissipate heat of the illumination module, so that the heat is dissipated using a large surface area of the heat dissipation chassis to prevent damage to a human body.

## Description

### [BACKGROUND]

### [Technical Field]

Embodiments of the present invention relate to an endoscope, and more particularly, to an endoscope in which an insertion unit and an operating unit are detachably provided so that a disposable insertion unit can be replaced and used.

### [Background Art]

A medical endoscope is a device used to check a condition of an affected area or perform a procedure by being directly inserted into internal body tissues of a subject for examination or surgery.

A typical endoscope includes an articulated assembly disposed at a distal end of a flexible insertion tube to allow an insertion direction to be adjusted during insertion into a human body. A distal end body equipped with an image sensor and a light source is provided at an end of the articulated assembly. A handle body may be provided at the other end of the insertion tube. The handle body is connected to an image processing device (not shown) through a separate cable and a connector.

Dials and buttons used for controlling the direction of the articulated assembly and performing procedures are disposed on the handle body.

A pair of dials are disposed to adjust a direction of travel of the distal end body relative to a longitudinal direction of the insertion tube in vertical and horizontal directions. A sprocket and a chain are provided inside the handle body to convert rotational movement of the dials into linear movement. The chain is connected to an end of the articulated assembly through a separate wire. As a result, rotation of the dials is converted into linear movement of the wire via the sprocket and the chain, and the articulated assembly is bent according to the linear movement of the wire. This allows an operator of the endoscope to adjust the direction of travel of the distal end body during insertion of the insertion tube into the human body.

Here, because the endoscope is inserted into the human body, thorough hygiene management is required, and cleaning/disinfection must be performed before and after examination or surgery. In some cases, it may be impossible or undesirable to reuse the portion actually inserted into the human body, that is, the insertion tube and the articulated assembly. In this case, since a conventional endoscope is formed of a single body entirely, a problem arises in that the entire endoscope apparatus must be replaced. Furthermore, even if reuse is possible, cleaning and disinfection can be performed more simply if only a part of the endoscope can be separated, rather than the entire endoscope.

Meanwhile, an LED may be provided in the distal end body, which is most likely to come into contact with the human body among the insertion portion, for illumination. In this case, there is a concern that heat generated from the LED may cause damage to the human body.

In this regard, Japanese Registered Patent No. JP4757358B2 discloses an endoscope having a heat dissipation structure.

However, the endoscope has a limitation in that a heat dissipation area is narrow because heat is only dispersed through a patterned metal, and a portion receiving the heat is also limited to the vicinity of an image sensor. Thus, there is a limitation in that heat from a plurality of heating elements, such as a PCB, an LED, and an image sensor, cannot all be dissipated.

### [DETAILED DESCRIPTION OF THE INVENTION]

### [Technical Problem]

Accordingly, one object of the embodiments of the present invention is to solve the above-noted disadvantages of the prior art, and to provide an endoscope capable of preventing damage to a human body by dissipating heat generated at a distal end of an insertion portion.

Further, another object of the present invention is to provide an endoscope that may prevent components disposed inside an illumination and imaging unit from being damaged by heat due to heat generated from a plurality of heating elements.

### [Technical Solution]

In order to achieve the above objects, an endoscope according to the present invention may include: an insertion unit having an illumination and imaging unit and an insertion tube; and an operating unit provided with an operating knob and configured to perform a bending operation of the insertion tube according to an operation of the operating knob, wherein the illumination and imaging unit may include: an illumination and imaging unit body; an illumination module configured to illuminate light; a heat sink in contact with the illumination module; and a heat dissipation chassis provided to surround an outer circumferential surface of the illumination and imaging unit body and disposed to contact the heat sink to dissipate heat of the illumination module.

Through this, there is an effect that heat can be dissipated using a large surface area of the heat dissipation chassis.

In this case, the heat sink may include: a heat sink body; and an illumination contact portion extending from one end in a longitudinal direction of the heat sink body and contacting the illumination module.

Through this, the heat sink can transfer the heat of the illumination module to the heat dissipation chassis to dissipate the heat.

Meanwhile, the illumination and imaging unit may further include an imaging module configured to image an image, and an imaging module receiving hole for accommodating the imaging module so as to pass therethrough may be formed in the heat sink body.

Through this, there is an effect that an area of the heat sink can be maximized within a limited space, and at the same time, heat of the imaging module can also be dissipated.

In addition, the heat sink may further include a heat transfer portion extending from the heat sink body in a width direction and contacting the heat dissipation chassis.

Meanwhile, the illumination module may include an illumination lens configured to illuminate light, and a plurality of the illumination lenses may be disposed.

In addition, the plurality of illumination lenses may be symmetrically disposed with respect to the imaging module.

Through this, asymmetrical illuminance can be prevented.

Meanwhile, the plurality of illumination lenses may have different Correlated Color Temperatures (CCT).

Through this, a target wavelength distribution of light can be created.

Meanwhile, the illumination and imaging unit may further include a heat shield plate configured to block heat of the illumination module.

In this case, the heat shield plate may include: a shield plate body formed in a semi-circular shape; and an illumination cover portion symmetrically disposed on the shield plate body and disposed to face the illumination module.

In addition, the heat shield plate may further include a camera cover portion disposed on an axis of symmetry of the pair of illumination cover portions and disposed at a position facing the imaging module.

Through this, heat can be prevented from being transferred to a human body from a portion where heat is mainly generated.

Meanwhile, the illumination and imaging unit may further include a printed circuit board configured to control the illumination module, and the heat sink may be in contact with the printed circuit board.

Through this, there is an effect that heat generated from the printed circuit board can also be dissipated.

### [Advantageous Effects]

As described above, according to the endoscope of the present invention, heat generated from an illumination module is transferred and can be dissipated using a large surface area of a heat dissipation chassis, thereby having an effect of preventing damage to a human body caused by the heat generated from the illumination module.

Along with this, since heat generated from an imaging module and a printed circuit board is also dissipated through a heat sink and a heat dissipation chassis, there is an effect of preventing components disposed inside an illumination and imaging unit from being damaged by heat according to the present invention.

### [Description of Drawings]

FIG. 1 is a perspective view for explaining an endoscope according to an embodiment of the present invention;
FIG. 2 is a perspective view for explaining a detachable relationship between an operating unit and an insertion unit in the endoscope according to an embodiment of the present invention;
FIG. 3 is a top view of FIG. 2;
FIG. 4 is a view for explaining a coupling method of a first coupling part and a second coupling part in the endoscope according to an embodiment of the present invention;
FIG. 5 is an enlarged view of FIG. 4 as viewed from another angle;
FIG. 6 is a view for explaining a state in which the first coupling part and the second coupling part are coupled in the endoscope according to an embodiment of the present invention;
FIGS. 7 to 13 are views for explaining a structure for guiding an initial position of a wire frame in the endoscope according to an embodiment of the present invention;
FIG. 14 is a perspective view of FIG. 2 as viewed from another angle;
FIG. 15 is a perspective view for explaining an illumination and imaging unit in the endoscope according to an embodiment of the present invention;
FIG. 16 is a perspective view for explaining an internal structure of the illumination and imaging unit in the endoscope according to an embodiment of the present invention e;
FIG. 17 is a view for explaining a heat sink in the endoscope according to an embodiment of the present invention;
FIGS. 18 and 19 are views for explaining an insertion tube in the endoscope according to an embodiment of the present invention;
FIG. 20 is a view for explaining a first bending member in the endoscope according to an embodiment of the present invention; and
FIG. 21 is a view for explaining a second bending member in the endoscope according to an embodiment of the present invention.

### [Description of Embodiments]

Hereinafter, preferred embodiments of the present invention will be described in detail with reference to the accompanying drawings.

The present invention may be subject to various changes and may have various embodiments, and specific embodiments will be illustrated in the drawings and described in detail in the detailed description. This is not intended to limit the present invention to specific embodiments, and it should be interpreted to include all changes, equivalents, and substitutes included in the spirit and technical scope of the present invention.

In describing the present invention, terms such as "first" and "second" may be used to describe various components, but the components may not be limited by the terms. These terms are only used for the purpose of distinguishing one component from another. For example, a first component may be named a second component without departing from the scope of rights of the present invention, and similarly, a second component may also be named a first component.

The term "and/or" may include a combination of a plurality of related listed items or any of the plurality of related listed items.

When a component is referred to as being "connected" or "coupled" to another component, it may be directly connected or coupled to the other component, but it should be understood that other components may exist in between. On the other hand, when a component is referred to as being "directly connected" or "directly coupled" to another component, it should be understood that no other components exist in between.

The terms used in the present application are only used to describe specific embodiments and are not intended to limit the present invention. Singular expressions may include plural expressions unless the context clearly indicates otherwise.

In the present application, terms such as "comprise" or "have" are intended to designate that features, numbers, steps, operations, components, parts, or combinations thereof described in the specification exist, and it should be understood that they do not preclude the presence or addition of one or more other features, numbers, steps, operations, components, parts, or combinations thereof.

Unless otherwise defined, all terms used herein, including technical or scientific terms, may have the same meaning as commonly understood by those of ordinary skill in the art to which the present invention belongs. Terms such as those defined in commonly used dictionaries may be interpreted as having a meaning consistent with the meaning in the context of the related technology, and unless explicitly defined in the present application, they may not be interpreted in an ideal or excessively formal sense.

In addition, the following embodiments are provided to more completely explain to those of ordinary skill in the art, and the shapes and sizes of elements in the drawings may be exaggerated for a clearer explanation.

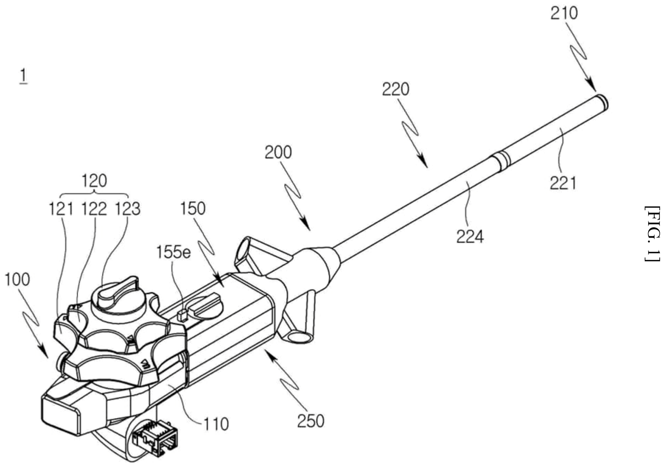

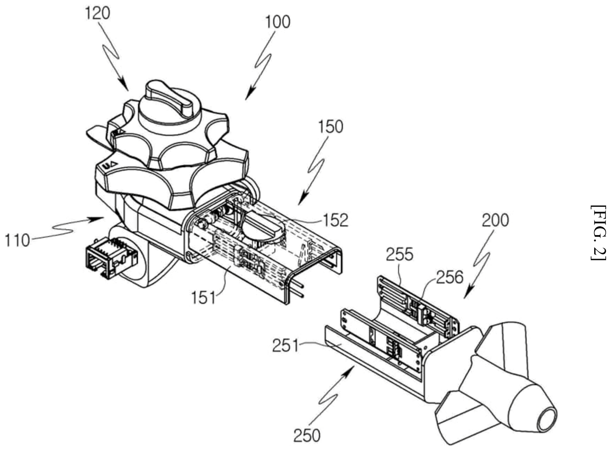

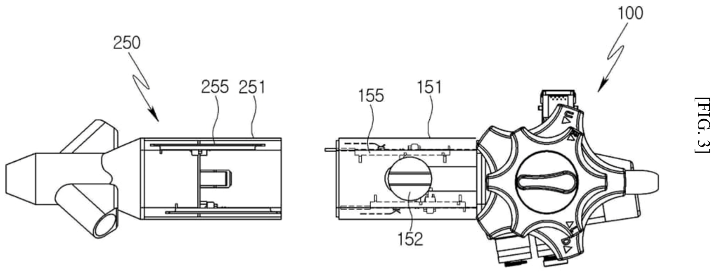

FIG. 1 is a perspective view for explaining an endoscope according to an embodiment of the present invention, FIG. 2 is a perspective view for explaining a detachable relationship between an operating unit and an insertion unit in the endoscope according to an embodiment of the present invention, and FIG. 3 is a top view of FIG. 2.

Referring to FIGS. 1 to 3, an endoscope 1 according to an embodiment of the present invention may be a disposable endoscope. Specifically, the endoscope 1 according to an embodiment of the present invention includes an operating unit 100 and an insertion unit 200. At this time, the operating unit 100 and the insertion unit 200 may be detachably coupled to each other by coupling parts 150 and 250. In this case, the coupling parts 150 and 250 include a first coupling part 150 provided in the operating unit 100 and a second coupling part 250 provided in the insertion unit 200.

The coupling parts 150 and 250 in the present invention are configured to convert a rotational movement of a dial 152 into a linear movement by a pinion gear 153 and a rack gear 154, so that coupling plates 155 and 255 provided in the operating unit 100 and the insertion unit 200 are coupled to each other. In this case, the coupling plates 155 and 255 include a first coupling plate 155 provided in the operating unit 100 and a second coupling plate 255 provided in the insertion unit 200.

Meanwhile, in the present invention, connection of wires 130 and 230 may be achieved by wire frames 156 and 256. That is, the wires 130 and 230 include an operating wire 130 provided in the operating unit 100 and a bending wire 230 provided in the insertion unit 200, and these are respectively coupled to a first wire frame 156 provided in the operating unit 100 and a second wire frame 256 provided in the insertion unit 200. At this time, the first wire frame 156 and the second wire frame 256 are detachably coupled to each other, and in a coupled state, they may linearly reciprocate within the coupling plates 155 and 255.

This will be described in detail later.

The operating unit 100 is provided so that an operator can operate it. The operating unit 100 may be provided so that an operator can grip it and operate it. The operating unit 100 may operate to bend the insertion unit 200 through an operator's operation.

The insertion unit 200 is inserted into a human body by operation of the operating unit 100 and is provided to image the inside of the human body. The insertion unit 200 may be inserted into the human body, may be provided to be bendable according to operation of the operating unit 100, may be moved along an internal structure of the human body, and may illuminate and image the inside of the human body.

Although not shown, the endoscope 1 according to an embodiment of the present invention may further include a connector adapter and a universal code.

Specifically, the endoscope 1 includes a connector adapter for connection to an external device, and the connector adapter may be connected to the operating unit 100 by a universal code. The external device connected to the connector adapter may be an image processing device. Through this, an image imaged by the insertion unit 200 may be displayed on the external image processing device. Through this, the operator can operate the operating unit 100 while watching the image imaged by the insertion unit 200 on the image processing device.

The operating unit 100 includes an operating unit housing 110, an operating knob 120, an operating wire 130, and a first coupling part 150.

The operating unit housing 110 provides a structure for a practitioner to grip it, and may accommodate an operating wire 130 and the like for driving the insertion unit 200 inside.

Specifically, an operating knob 120 is disposed at an upper portion of the operating unit housing 110, a first coupling part 150 is disposed at one side in a longitudinal direction of the operating knob 120, and a sprocket and a chain linked to rotation of the operating knob 120 and an operating wire 130 are accommodated inside the operating unit housing 110, and a channel and the like may be further accommodated therein.

The operating knob 120 may control the insertion unit 200 while rotating by an operator's operation. The operating knob 120 may include two or more knobs. For example, the operating knob 120 may be provided in a form where a first knob 121 and a second knob 122 are stacked along a vertical direction. The first knob 121 and the second knob 122 are respectively connected to a first sprocket and a second sprocket (which will be described later), and configured to be able to rotate independently of each other. The first knob 121 and the second knob 122 have a shape in which a plurality of teeth protrude so that a practitioner can easily grip and rotate them.

Meanwhile, the first knob 121 may rotate independently while the second knob 122 maintains a stopped state. Similarly, the second knob 122 may rotate independently while the first knob 121 maintains a stopped state. The first knob 121 controls vertical movement of the illumination and imaging unit 210. That is, when the first knob 121 is rotated in a clockwise or counterclockwise direction, a distal end of the illumination and imaging unit 210 is bent upward or downward accordingly. The second knob 122 controls horizontal movement of the illumination and imaging unit 210. That is, when the second knob 122 is rotated in a clockwise or counterclockwise direction, the distal end of the illumination and imaging unit 210 is bent to the left or right accordingly.

By operating the first knob 121 and the second knob 122 in this way, the rotation direction of the illumination and imaging unit 210 can be arbitrarily adjusted. According to an embodiment, there may be a need to fix the illumination and imaging unit 210 in a state of being bent at a specific angle, and for this purpose, a fixing knob 123 is provided above the second knob 122. When the fixing knob 123 is rotated, the first sprocket and the second sprocket are fixed so as not to rotate, and accordingly, the distal end of the illumination and imaging unit 210 also maintains a fixed state.

Although not shown, two sprockets are rotatably mounted below the first knob 121 and the second knob 122 in linkage with rotation of the first knob 121 and the second knob 122. And a chain is provided which moves in engagement with the respective sprockets. The chains are responsible for vertical movement and horizontal movement of the illumination and imaging unit 210, respectively, and they can move independently of each other.

Both ends of the chain are coupled to the operating wire 130, respectively. Movement of the operating wire 130 is guided through a guide structure formed inside the operating unit housing 110.

The operating wire 130 is coupled to the first coupling part 150. Specifically, the operating wire 130 is coupled by the first wire frame 156 of the first coupling part 150. The first wire frame 156 is slidably mounted within a limited range of the first coupling plate 155.

Meanwhile, the first wire frame 156 is coupled to the second wire frame 256 of the insertion unit 200, whereby the operating wire 130 can be connected to the bending wire 230.

Accordingly, the operating wire 130 can perform a bending operation of the insertion tube 220 according to the operation of the operating knob 120.

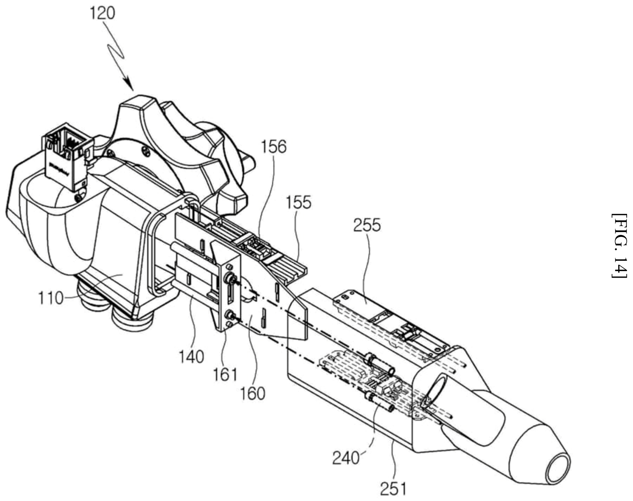

Meanwhile, FIG. 14 is a perspective view of FIG. 2 as viewed from another angle.

Referring to FIG. 14, the operating unit 100 may be provided with a channel 140 for providing air and water to an affected area during a procedure. For example, the channel 140 may be a supply channel and a suction channel. In this case, the supply channel may be an air supply channel and/or a water supply channel. The air supply channel, the water supply channel, and the suction channel have a substantially cylindrical shape and have an empty interior, so that when coupled with the illumination and imaging unit 210, they are inserted into the illumination and imaging unit 210 to ensure that water, air, or the like can be stably supplied to the procedure or examination site without leakage.

Meanwhile, although not shown, the operating unit 100 may further include a connection terminal. The connection terminal may be provided to be electrically connected to the illumination and imaging unit 210 so that data obtained by a camera or the like provided at a distal end of the illumination and imaging unit 210 can be transmitted to an external device. For example, the connection terminal may be an HDMI terminal for transmitting audio and video.

The channel 140 and the connection terminal may be disposed below the wires 130 and 230. For example, the channel 140 and the connection terminal are accommodated in a space formed of the operating unit housing 110, the first coupling part body 151, and the second coupling part body 251, but may be disposed further from the operating knob 120 than the operating wire 130.

At this time, a region where the operating wire 130 and the bending wire 230 move and a region where the channel 140 and the connection terminal are disposed may be divided by a support plate 160. The support plate 160 may partition an internal space of the operating unit 100 into upper and lower parts, and may include a channel support part 161 coupled with the channel 140 to support the channel 140, thereby guiding a fixed-position connection of the channel 140 when the operating unit 100 and the insertion unit 200 are coupled. That is, when the first coupling part 150 and the second coupling part 250 are coupled, the channel 140 of the operating unit 100 may be connected to the channel 240 of the insertion unit 200.

The channel support part 161 is formed to extend downward from a lower surface of the support plate 160, and a circular hole may be formed such that the channel 140 passes therethrough to be supported. In this case, the number of the holes may be formed to correspond to the number of the channels 140.

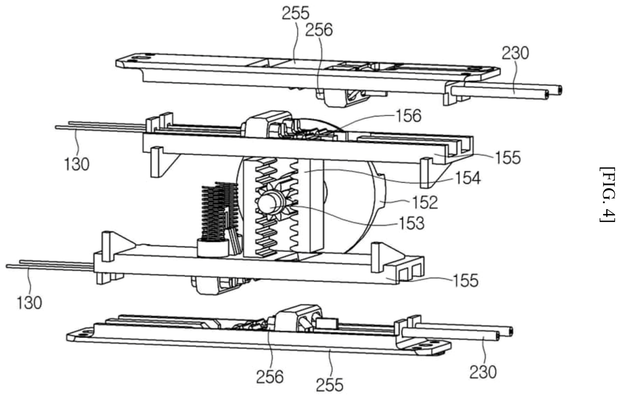

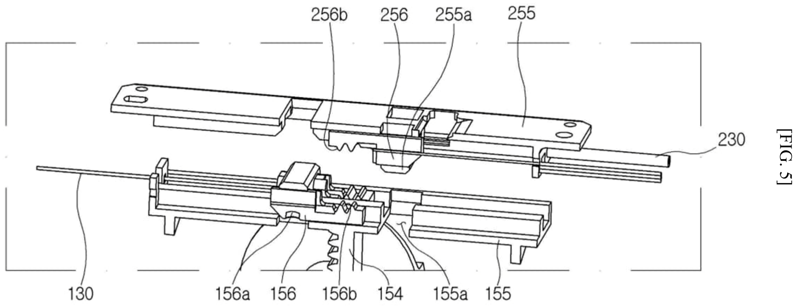

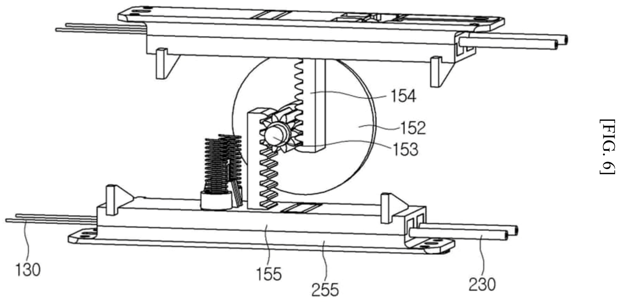

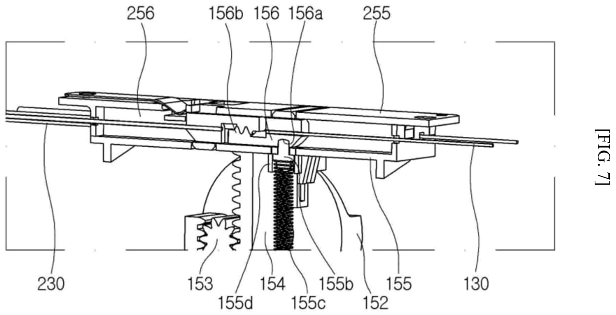

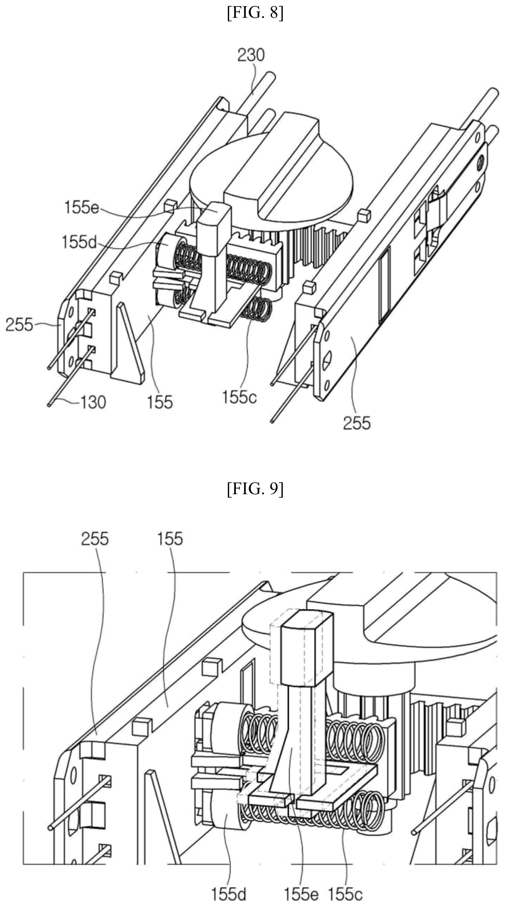

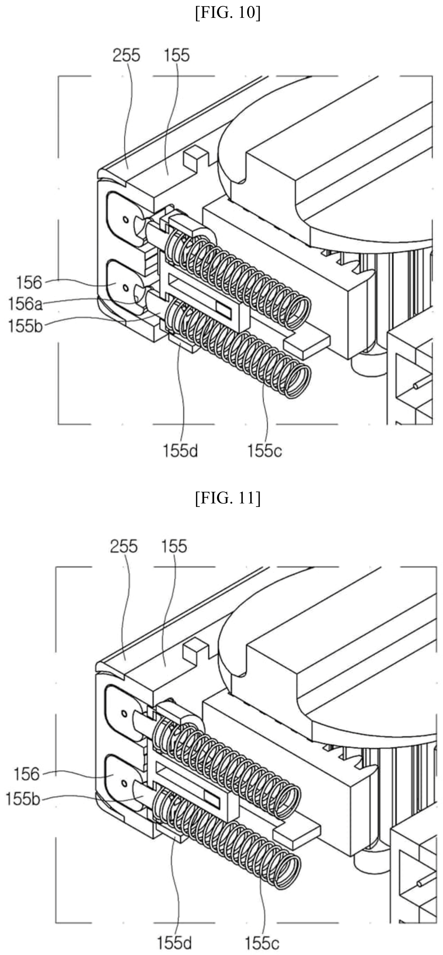

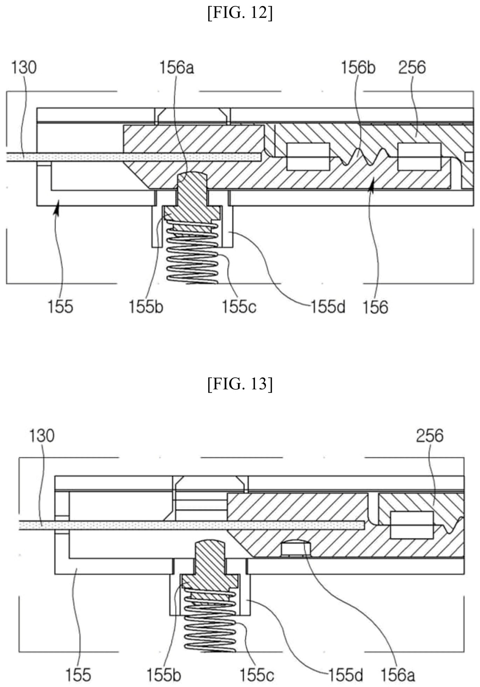

Meanwhile, FIG. 4 is a view for explaining a coupling method of a first coupling part and a second coupling part in the endoscope according to an embodiment of the present invention, FIG. 5 is an enlarged view of FIG. 4 as viewed from another angle, FIG. 6 is a view for explaining a state in which the first coupling part and the second coupling part are coupled in the endoscope according to an embodiment of the present invention, and FIGS. 7 to 13 are views for explaining a structure for guiding an initial position of a wire frame in the endoscope according to an embodiment of the present invention.

Referring to FIGS. 4 to 13, the first coupling part 150 is described as follows.

The first coupling part 150 may be disposed at one end of the operating unit housing 110. The first coupling part 150 may be detachably coupled to the insertion unit 200 by an operation of a practitioner (user).

Specifically, the first coupling part 150 includes a first coupling part body 151, a dial 152, a pinion gear 153, a rack gear 154, and a first coupling plate 155.

The first coupling part body 151 is provided to engage and couple with the second coupling part 250 of the insertion unit 200. For example, the first coupling part body 151 may be a plate shape wrapping around three sides. As another example, the first coupling part body 151 may be a pair of plate shapes extending from the operating unit housing 110 in a direction of the insertion unit 200. Accordingly, the first coupling part body 151 may be coupled with the second coupling part body 251 of the second coupling part 250.

As a result, the first coupling part body 151 and the second coupling part body 251 may be fitted into each other.

The dial 152 may be provided so that a user can grip and rotate it. The dial 152 may be rotatably coupled to the operating unit housing 110.

Specifically, the dial 152 may be exposed to the outside of the operating unit housing 110. For example, the dial 152 may include a disc-shaped rotating plate and a gripping part which protrudes in a rib shape from the rotating plate so that a user can hold and rotate it.

The pinion gear 153 is disposed inside the first coupling part body 151, and may be rotated in linkage with the rotation of the dial 152.

Meanwhile, the pinion gear 153 may be connected to the dial 152. The pinion gear 153 may be connected to the dial 152 by passing through the first coupling part body 151. For example, the pinion gear 153 and the dial 152 may be formed integrally.

Gear teeth are formed on an outer circumferential surface of the pinion gear 153, and may be engaged with a rack gear 154 (which will be described later). At this time, the pinion gear 153 may be engaged with a pair of rack gears 154. The pair of rack gears 154 may be disposed at positions facing each other with respect to the pinion gear 153.

The rack gear 154 is engaged with the pinion gear 153, and may be linearly moved according to the rotation of the pinion gear 153. At this time, the pair of rack gears 154 facing each other may be linearly moved in opposite directions according to the rotation of the pinion gear 153.

The pair of rack gears 154 may be disposed between the pair of first coupling part bodies 151. In this case, the pair of rack gears 154 may be disposed along a direction intersecting a direction in which the first coupling part body 151 is formed, and may be linearly reciprocated along a direction intersecting the direction in which the first coupling part body 151 is formed.

The pair of rack gears 154 may be respectively coupled to the first coupling plate 155. The first coupling plate 155 may be formed along a direction intersecting a longitudinal direction of the rack gear 154.

The first coupling plate 155 may be disposed between a pair of surfaces facing each other among the first coupling part bodies 151. And, the first coupling plate 155 may be disposed between the pair of second coupling part bodies 251. Meanwhile, the first coupling plate 155 may be disposed inside the second coupling part body 251, and disposed at a position facing the second coupling part body 251.

In addition, the first coupling plate 155 may be disposed at a position facing the second coupling plate 255. The first coupling plate 155 is moved together with the rack gear 154 according to the movement of the rack gear 154, and may be coupled with the second coupling plate 255.

For example, the first coupling plate 155 may be formed in a square plate shape having a predetermined thickness. In this case, a fixing part 155a for fixing the coupling with the second coupling plate 255 may be formed on the first coupling plate 155. For example, the fixing part 155a may be formed in a hole shape and coupled with a protrusion-shaped fixing part 255a formed on the second coupling plate 255.

Meanwhile, the first wire frame 156 may be disposed on the first coupling plate 155. The first wire frame 156 may be coupled to the first coupling plate 155 so as to be linearly reciprocable. For example, a guide groove for guiding the linear movement of the first wire frame 156 may be formed in the first coupling plate 155, and the first wire frame 156 may be disposed in the guide groove.

Meanwhile, the operating wire 130 is coupled to the first wire frame 156. For example, the operating wire 130 is coupled to one side in a longitudinal direction of the first wire frame 156, and may be provided to be linearly reciprocable along the longitudinal direction.

Meanwhile, the first wire frame 156 disposed on the first coupling plate 155 may be coupled to the second wire frame 256 disposed on the second coupling plate 255. For example, a frame coupling part 156b may be formed at the other side in the longitudinal direction of the first wire frame 156. The frame coupling part 156b may have a shape in which a plurality of ribs and grooves are alternately formed. The frame coupling part 156b may be formed in a shape corresponding to a frame coupling part 256b of the second wire frame 256. That is, the first wire frame 156 and the second wire frame 256 may be coupled in an engaged state with each other.

The first wire frame 156 may be disposed between the first coupling plate 155 and the second coupling plate 255 in a state coupled with the second wire frame 256. Therefore, the first wire frame 156 and the second wire frame 256, in the coupled state, can linearly reciprocate along the longitudinal direction of the endoscope 1.

Through this, the operating wire 130 coupled to the first wire frame 156 and the bending wire 230 coupled to the second wire frame 256 can move in linkage with each other, and the insertion tube 220 can be bent according to the operation of the operating knob 120.

Meanwhile, the first coupling plate 155 may further include a locking pin 155b, a spring 155c, and a locking holder 155d.

The locking pin 155b is disposed on the first coupling plate 155, and may be accommodated in a fixing groove 156a formed in the first wire frame 156 according to reciprocating movement. The locking pin 155b may include a head part accommodated in a locking holder 155d (which will be described later) and a pin part accommodated in the fixing groove 156a by passing through the locking holder 155d. At this time, the head part may be formed in a shape having a diameter larger than that of the pin part. Accordingly, a range of movement of the locking pin 155b may be limited by the locking holder 155d.

Meanwhile, the locking pin 155b may be biased toward the first wire frame 156 through the spring 155c. Specifically, the head part of the locking pin 155b may contact one end of the spring 155c. Accordingly, the locking pin 155b may be elastically supported by the spring 155c.

The other end of the spring 155c may be fixed to the first coupling part body 151.

The locking holder 155d is detachably coupled to the first coupling plate 155, and may accommodate the locking pin 155b so as to be reciprocable.

The locking holder 155d is coupled with a locking lever 155e, and may be reciprocated by an operation of the locking lever 155e. At this time, the locking holder 155d may be detachably coupled to the first coupling plate 155 according to the operation of the locking lever 155e.

The locking holder 155d may be formed to accommodate the locking pin 155b therein. For example, the locking holder 155d is formed in a cylindrical shape, wherein one side in a longitudinal direction may be formed corresponding to a size of the head part of the locking pin 155b, and the other side in the longitudinal direction may be formed corresponding to a size of the pin part of the locking pin 155b. That is, one side in the longitudinal direction of the locking holder 155d may be formed to be larger than a diameter of the head part, and the other side in the longitudinal direction may be formed to be smaller than the diameter of the head part but larger than a diameter of the pin part.

Therefore, the pin part of the locking pin 155b may pass through the locking holder 155d and be accommodated in the fixing groove 156a of the first wire frame 156.

For example, a pair of locking holders 155d may be provided, and a lever coupling part coupled with the locking lever 155e may be provided between the pair of locking holders 155d. The lever coupling part may be formed in a long hole shape so that one side of the locking lever 155e can be coupled thereto.

Meanwhile, one side of the locking lever 155e is coupled to the locking holder 155d, and the other side may be exposed to the outside of the first coupling part body 151. Accordingly, a user can hold and operate the locking lever 155e.

Meanwhile, a fixing groove 156a may be formed in the first wire frame 156. The fixing groove 156a may be formed on a surface opposite to a surface on which the frame coupling part 156b is formed. That is, the frame coupling part 156b may be formed on a surface at a position facing the second wire frame 256, and the fixing groove 156a may be formed on the opposite surface thereof.

The fixing groove 156a may accommodate the locking pin 155b according to movement of the first wire frame 156. That is, when the first wire frame 156 is disposed at a preset initial position, the fixing groove 156a may be disposed to face the locking pin 155b. At this time, when a user operates the locking lever 155e, the locking pin 155b is moved along the locking holder 155d and may be inserted into the fixing groove 156a by passing through the first coupling plate 155.

Therefore, according to the present invention, after replacing the insertion unit 200, the user can rotate the operating knob 120 while the locking lever 155e is operated, and accordingly, a position where the locking pin 155b and the fixing groove 156a are coupled can be found as the first wire frame 156 linearly moves (slidably moves).

As a result, in the present invention, there is an effect that an initial position of the operating wire 130 can be found and maintained after the insertion unit 200 is replaced.

The insertion unit 200 is detachably coupled to the operating unit 100, and at least a portion thereof is inserted into a human body to image the inside of the human body.

The insertion unit 200 includes an illumination and imaging unit 210, an insertion tube 220, a bending wire 230, and a second coupling part 250. At this time, the illumination and imaging unit 210 is disposed at one side in a longitudinal direction of the insertion unit 200, and the second coupling part 250 is disposed at the other side thereof. In addition, the illumination and imaging unit 210 and the second coupling part 250 may be connected by the insertion tube 220, and at least a portion of the bending wire 230 may pass from the second coupling part 250 to the insertion tube 220.

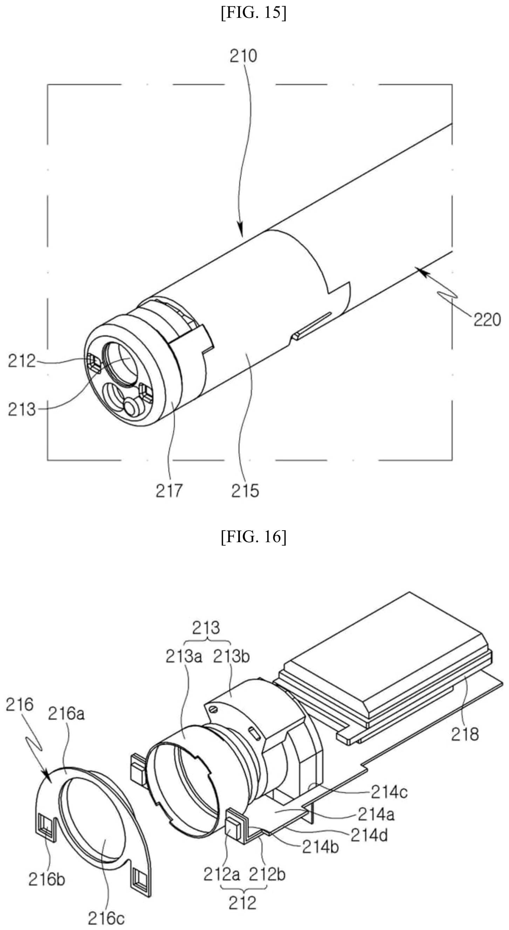

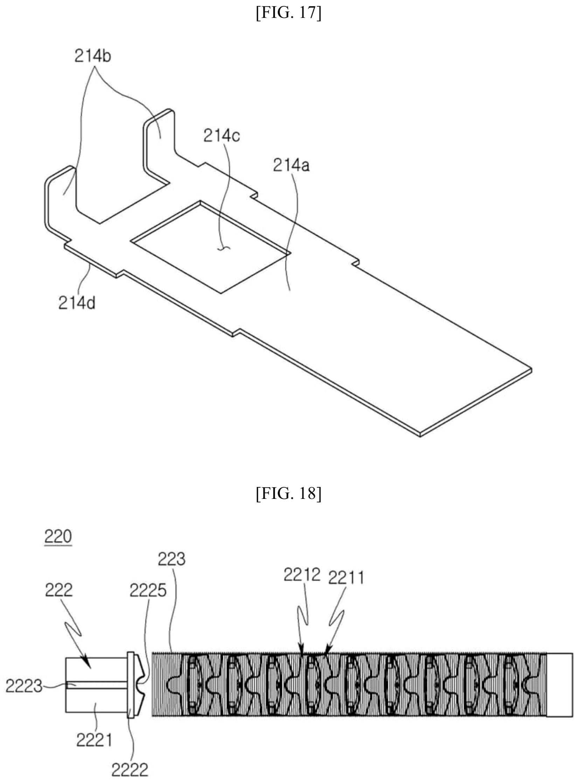

Meanwhile, FIG. 15 is a perspective view for explaining an illumination and imaging unit in the endoscope according to an embodiment of the present invention, FIG. 16 is a perspective view for explaining an internal structure of the illumination and imaging unit in the endoscope according to an embodiment of the present invention, and FIG. 17 is a view for explaining a heat sink in the endoscope according to an embodiment of the present invention.

The illumination and imaging unit 210 is described as follows with reference to FIGS. 15 to 17.

The illumination and imaging unit 210 is inserted into a human body to illuminate the inside of the human body and image the inside of the human body.

The illumination and imaging unit 210 includes an illumination and imaging unit body 211, an illumination module 212, and an imaging module 213.

The illumination and imaging unit body 211 forms an exterior of the illumination and imaging unit 210 and is configured to be inserted into the human body. For example, the illumination and imaging unit body 211 may have an illumination module 212 and an imaging module 213 disposed at one side in a longitudinal direction, and the other side in the longitudinal direction may be open to be connected to the insertion tube 220.

The illumination module 212 may be coupled to an end cap 217. The illumination module 212 can illuminate light. The illumination module 212 may include an illumination lens 212a capable of illuminating light upon receiving power. For example, the illumination lens 212a may be an LED (Light Emitting Diode).

Meanwhile, in the present embodiment, a plurality of illumination lenses 212a may be provided. Specifically, in the present embodiment, an even number of illumination lenses 212a may be provided. For example, a pair of illumination lenses 212a may be disposed line-symmetrically. This has an effect of preventing illuminance on an object from being asymmetrical at a short distance and preventing a gap in illumination from occurring.

At this time, in the present embodiment, the pair of illumination lenses 212a may be symmetrically disposed with respect to the imaging module 213. That is, in the present embodiment, the pair of illumination lenses 212a may be symmetrically disposed, and the imaging module 213 may be disposed on an axis of symmetry.

In this case, the pair of illumination lenses 212a may have different Correlated Color Temperatures (CCT). Through this, a target wavelength distribution of light can be created.

In addition, the illumination module 212 may further include an illumination support part 212b supporting the pair of illumination lenses 212a. The illumination support part 212b is formed in a flat plate shape, wherein a pair of distal ends are symmetrically extended and bent upward. The pair of illumination lenses 212a may be coupled to the pair of bent extended parts.

For example, the illumination support part 212b may be a flexible printed circuit board (FPCB). An image sensor may be mounted on the FPCB. Since a plurality of illumination lenses 212a are utilized as an illumination part, not only is it easy to adjust illuminance, but also an amount of irradiated light can be significantly increased compared to conventional halogen lamps or the like, thereby further improving clarity of an image obtained through the image sensor.

Meanwhile, the imaging module 213 can image the inside of a human body. The imaging module 213 may image an image of the inside of the human body upon receiving power and transmit captured image data. For example, the imaging module 213 may include a camera 213a and an image processor 213b. In this case, the camera 213a may be disposed at one end (distal end) in the longitudinal direction of the illumination and imaging unit 210, and the image processor 213b may be coupled with the camera 213a to process an image received from the camera.

Through this, the endoscope 1 of the present invention can illuminate light through the illumination module 212 while being inserted into the human body and image the inside of the human body through the imaging module 213.

The heat sink 214 may be disposed inside the illumination and imaging unit body 211. The heat sink 214 may be coupled with the illumination module 212 and/or the imaging module 213.

The heat sink 214 includes a heat sink body 214a, an illumination contact part 214b, an imaging module receiving hole 214c, and a heat transfer part 214d.

The heat sink body 214a may be provided to transfer heat. The heat sink body 214a may be formed of a material capable of transferring heat. For example, the heat sink body 214a may be formed of a metal material.

The heat sink body 214a is formed in a flat plate shape, wherein one end in a longitudinal direction (long-axis direction) is connected to the illumination contact part 214b, heat transfer parts 214d are connected to both sides in a width direction (short-axis direction), and an imaging module receiving hole 214c may be formed therein.

The illumination contact part 214b is formed to extend from one end in the longitudinal direction of the heat sink body 214a and may contact the illumination module 212. The illumination contact part 214b may be formed corresponding to a shape of the illumination support part 212b. For example, the illumination contact part 214b may be formed to extend from a distal end in the longitudinal direction of the heat sink body 214a and then bent and extend upward. Accordingly, the illumination contact part 214b may contact the illumination support part 212b. Through this, heat generated from the illumination module 212 can be transferred to the heat sink 214 via the illumination contact part 214b.

The imaging module receiving hole 214c is formed in the heat sink body and may accommodate the imaging module 213 so as to pass therethrough. For example, the imaging module receiving hole 214c may be formed in a rectangular hole shape, where a portion of the image processor 213b passes therethrough and another portion of the image processor 213b may be in contact. Through this, heat generated from the imaging module 213 can be transferred to the heat sink 214.

Meanwhile, a printed circuit board 218 may contact the other end in the longitudinal direction of the heat sink 214. The printed circuit board 218 may control the illumination module 212 and/or the imaging module 213. A circuit capable of controlling the illumination module 212 and/or the imaging module 213 may be mounted on the printed circuit board 218. Through this, heat generated from the circuit can be transferred to the heat sink 214.

The heat transfer part 214d may be formed to extend from both ends in a width direction (short-axis direction) of the heat sink body 214a. At this time, a width of the heat sink body 214a is smaller than a diameter of the heat dissipation chassis 215, and a combined width of the heat sink body 214a and the heat transfer part 214d may be equal to or slightly larger than the diameter of the heat dissipation chassis 215. Therefore, the heat transfer part 214d may contact the heat dissipation chassis 215. Accordingly, heat of the heat transfer part 214d can be transferred to the heat dissipation chassis 215.

The heat dissipation chassis 215 is provided to wrap around an outer circumferential surface of the illumination and imaging unit body 211 and contacts the heat sink 214 to dissipate heat of the illumination module 212 and/or the imaging module 213.

For example, the heat dissipation chassis 215 may be a coil pipe made of a spring material and can release heat generated from the illumination module 212 and/or the imaging module 213.

Meanwhile, in another embodiment of the present invention, the heat dissipation chassis 215 and the illumination and imaging unit body 211 may be formed integrally.

Meanwhile, the illumination and imaging unit 210 may further include a heat shield plate 216 that is disposed at one side in the longitudinal direction of the illumination and imaging unit 210 and blocks heat generated from the illumination module 212.

The heat shield plate 216 is coupled to the end cap 217, and may be disposed between the end cap 217 and the illumination module 212 along a longitudinal direction of the illumination and imaging unit 210.

The heat shield plate 216 may include a shield plate body 216a formed in a semi-circular shape, an illumination cover part 216b symmetrically disposed on the shield plate body 216a and disposed to face the illumination module 212, and a camera cover part 216c disposed on an axis of symmetry of the pair of illumination cover parts 216b and disposed at a position facing the imaging module 213.

The heat shield plate 216 may prevent heat generated from the illumination module 212 from being transferred to a human body. In this case, the heat shield plate 216 may be formed of a material through which light can pass.

An end cap 217 is provided at a longitudinal end (distal end) of the illumination and imaging unit 210. The end cap 217 has a cylindrical structure and is coupled to an end of the insertion unit 200. In this case, the illumination module 212 and the imaging module 213 may be coupled to the end cap 217. In addition, the heat shield plate 216 may be coupled to the end cap 217.

The end cap 217 may be provided with light transmission windows at a plurality of points. The light transmission window is made of a plate material of glass or a transparent material, and has a structure capable of transferring light irradiated from the illumination module 212 to the outside.

Meanwhile, the end cap 217 may be formed with a plurality of channel receiving tubes into which the channel 240 is inserted. The inside of each channel receiving tube has a hollow cylindrical shape and is formed to have an inner diameter corresponding to an outer diameter of each insertion channel 140. And, an O-ring for preventing leakage of supplied water or air may be provided inside.

Meanwhile, a connection terminal may be provided in the illumination and imaging unit 210. The connection terminal may be provided corresponding to the connection terminal of the operating unit 100. That is, a male terminal may be provided in the operating unit 100, and a female terminal may be provided in the insertion unit 200.

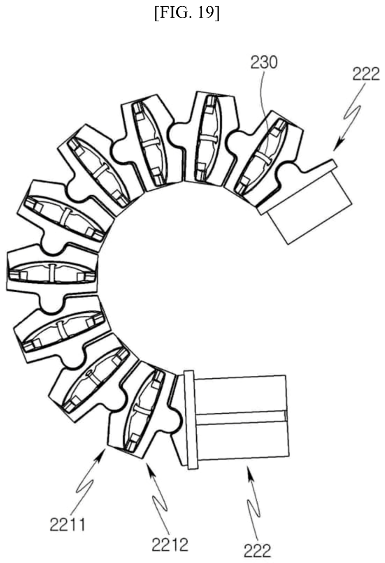

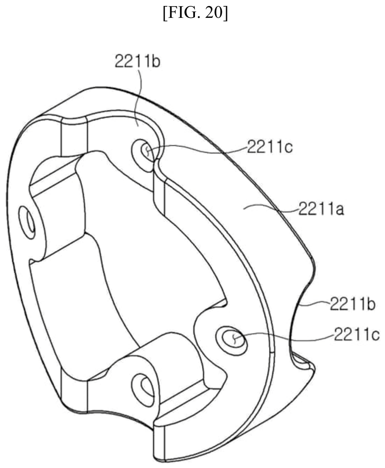

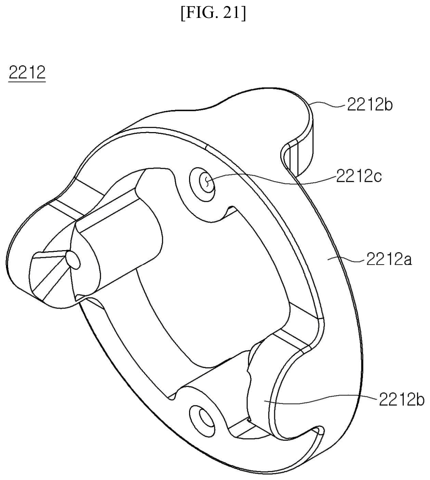

Meanwhile, FIGS. 18 and 19 are views for explaining an insertion tube in an endoscope according to an embodiment of the present invention, FIG. 20 is a view for explaining a first bending member in the endoscope according to an embodiment of the present invention, and FIG. 21 is a view for explaining a second bending member in the endoscope according to an embodiment of the present invention.

Referring to FIGS. 18 to 21, the insertion tube 220 is described as follows.

The insertion tube 220 connects the illumination and imaging unit 210 and the second coupling part 250, and may be bent according to an operation of the operating unit 100.

Specifically, the insertion tube 220 may be bent according to movement of the bending wire 230.

Meanwhile, a plurality of channels 240 and terminals may be accommodated inside the insertion tube 220.

The insertion tube 220 may include a bending part 221, an adapter 222, a coil spring 223, and an insertion tube 224. In this case, the bending part 221 is disposed between a pair of adapters 222, wherein one of the pair of adapters 222 may be coupled to the illumination and imaging unit 210, and the other may be coupled to the second coupling part 250.

The bending part 221 may be configured to be bent according to movement of the bending wire 230.

The bending part 221 includes a first bending member 2211 and a second bending member 2212 that are rotated relative to each other. In this case, the bending part 221 may have a plurality of first bending members 2211 and second bending members 2212 alternately disposed. As the number of the first bending members 2211 and the second bending members 2212 increases, the total length of the bending part 221 may increase. Therefore, an appropriate length of the insertion tube 220 can be selected by adjusting the number of the first bending members 2211 and the second bending members 2212 according to usage conditions of the endoscope 1.

The first bending member 2211 and the second bending member 2212 may be provided to be rotated relative to each other.

The first bending member 2211 includes a first bending member body 2211a, a concave part 2211b, and a wire through-hole 2211c.

The first bending member body 2211a may be formed in an overall annular shape. In this case, a thickness in a longitudinal direction of the first bending member body 2211a may repeatedly change at predetermined angular intervals along a circumferential direction. For example, the first bending member body 2211a may be formed in a shape in which the thickness decreases as it moves away from the concave part 2212b along the circumferential direction.

Meanwhile, in the present embodiment, the first bending member body 2211a may be formed of a resin material. For example, the first bending member body 2211a may be formed of a plastic material. With such a material, there are advantages in that processability is improved and production costs can be reduced.

At this time, a concave part 2211b may be formed in the first bending member body 2211a so as to be rotated relative to the second bending member 2212.

Four concave parts 2211b are formed in the first bending member body 2211a, wherein a pair may be formed at one longitudinal end of the first bending member body 2211a, and another pair may be formed at the other longitudinal end of the first bending member body 2211a. In this case, the pair formed at one end and the pair formed at the other end of the first bending member body 2211a may be alternately disposed with a phase difference of 90 degrees from each other along a circumferential direction. That is, a pair of concave parts 2211b formed at an end in the same direction are disposed with a phase difference of 180 degrees along the circumferential direction, but may be disposed with a phase difference of 90 degrees with respect to a pair formed at an end in the other direction.

The concave part 2211b may be recessed from a longitudinal end of the first bending member body 2211a. At this time, the concave part 2211b may be recessed in an arch shape having a predetermined curvature from the longitudinal end of the first bending member body 2211a.

The concave part 2211b may accommodate at least a portion of a convex part 2212b of the second bending member 2212, which will be described later. In addition, the concave part 2211b and the convex part 2212b may be rotated relative to each other. In this case, the concave part 2211b and the convex part 2212b may be in contact with each other.

Accordingly, in a state where the first bending member body 2211a and the second bending member body 2212a are coupled, a gap between the first bending member body 2211a and the second bending member body 2212a may not be constant. That is, when the first bending member 2211 and the second bending member 2212 are coupled, the concave part 2211b and the convex part 2212b may contact each other, and the gap between the first bending member body 2211a and the second bending member body 2212a may increase as it moves away from the concave part 2211b and the convex part 2212b along the circumferential direction.

Through this gap, a range in which the first bending member 2211 and the second bending member 2212 can be rotated relative to each other according to movement of the bending wire 230 may be provided.

Meanwhile, the first bending member body 2211a may have an increased radial thickness at a position where the concave portion 2211b is formed. For example, the first bending member body 2211a may be formed to have a constant outer diameter on a concentric circle, but an inner diameter at the position where the concave portion 2211b is formed may be formed to be smaller. That is, the first bending member body 2211a may be formed in a shape in which an inner circumferential surface at the position where the concave portion 2211b is formed protrudes radially inward.

Along with this, a wire through-hole 2211c may be formed along a longitudinal direction at the position where the concave portion 2211b is formed. That is, the wire through-hole 2211c may be a hole formed in a circular shape at a position protruding radially inward among the inner circumferential surface of the first bending member body 2211a.

At this time, the bending wire 230 may pass through the wire through-hole 2211c.

The second bending member 2212 includes a second bending member body 2212a, a convex portion 2212b, and a wire through-hole 2212c.

The second bending member body 2212a may be formed in an overall annular shape. In this case, a thickness in a longitudinal direction of the second bending member body 2212a may repeatedly change at intervals of 180 degrees along a circumferential direction. For example, the second bending member body 2212a may be formed such that the longitudinal thickness decreases as it moves away from a position where the convex portion 2212b is formed along the circumferential direction.

Meanwhile, in the present embodiment, the second bending member body 2212a may be formed of a resin material. For example, the second bending member body 2212a may be formed of a plastic material. With such a material, there are advantages in that processability is improved and production costs can be reduced.

At this time, a convex portion 2212b may be formed in the second bending member body 2212a so as to be rotated relative to the first bending member 2211.

Four convex portions 2212b are formed in the second bending member body 2212a, wherein a pair may be formed at one longitudinal end of the second bending member body 2212a, and another pair may be formed at the other longitudinal end of the second bending member body 2212a. In this case, the pair formed at one end and the pair formed at the other end of the second bending member body 2212a may be alternately disposed with a phase difference of 90 degrees from each other along a circumferential direction. That is, a pair of convex portions 2212b formed at an end in the same direction are disposed with a phase difference of 180 degrees along the circumferential direction, but may be disposed with a phase difference of 90 degrees with respect to a pair formed at an end in the other direction.

The convex portion 2212b may be formed to protrude from a longitudinal end of the second bending member body 2212a. At this time, the convex portion 2212b may be formed corresponding to a shape of the concave portion 2211b. For example, the convex portion 2212b may be formed to protrude in an arch shape having a predetermined curvature from the longitudinal end of the second bending member body 2212a, and a protruding curvature of the convex portion 2212b and a recessed curvature of the concave portion 2211b may be identical.

With this configuration, at least a portion of the convex portion 2212b may be accommodated in the concave portion 2211b. In addition, the convex portion 2212b and the concave portion 2211b may be rotated relative to each other while being in surface contact with each other.

Meanwhile, the second bending member body 2212a may have an increased radial thickness at a position where the convex portion 2212b is formed. For example, the second bending member body 2212a may be formed to have a constant outer diameter on a concentric circle, but an inner diameter at the position where the convex portion 2212b is formed may be formed to be smaller. That is, the second bending member body 2212a may be formed in a shape in which an inner circumferential surface at the position where the convex portion 2212b is formed protrudes radially inward.

Along with this, a wire through-hole 2212c may be formed along a longitudinal direction at the position where the convex portion 2212b is formed. That is, the wire through-hole 2212c may be a hole formed in a circular shape at a position protruding radially inward among the inner circumferential surface of the second bending member body 2212a.

At this time, the bending wire 230 may pass through the wire through-hole 2212c.

Meanwhile, an inner circumferential surface of the convex portion 2212b itself does not protrude radially inward, and may be formed identically to a maximum inner diameter of the second bending member body 2212a. That is, the inner circumferential surface of the convex portion 2212b does not protrude radially inward further than a position where the wire through-hole 2212c is formed. Therefore, when the inner circumferential surface of the second bending member 2212 is viewed entirely, it has a uniform inner circumferential surface, but the inner circumferential surface at the position where the wire through-hole 2212c is formed has a shape protruding radially inward.

Along with this, a wire guide groove 2212d may be formed in the inner circumferential surface of the convex portion 2212b. The wire guide groove 2212d is recessed in an arc shape in the inner circumferential surface of the convex portion 2212b, and an origin of the arc may be connected to the wire through-hole 2212c.

Due to this shape, when the bending wire 230 moves with a position contacting the second bending member body 2212a as a fixed end, a moving range of a free end may be limited by the wire guide groove 2212d. That is, a maximum angle of bending in each second bending member 2212 may be a center angle of the arc in the wire guide groove 2212d.

The adapters 222 may be respectively coupled to both ends in the longitudinal direction of the bending part 221. At this time, the adapter 222 disposed at one side in the longitudinal direction may be coupled to the illumination and imaging unit 210, and the adapter 222 disposed at the other side in the longitudinal direction may be coupled to the second coupling part 250.

The adapter 222 includes an adapter body 2221, a spring coupling part 2222, a wire receiving groove 2223, a wire through-hole, and a bending part connection part 2225.

The adapter body 2221 is formed in an overall cylindrical shape, and a spring coupling part 2222 may be formed on an outer circumferential surface of the adapter body 2221. In addition, a wire receiving groove 2223 may be formed along the longitudinal direction on an inner circumferential surface or the outer circumferential surface of the adapter body 2221. In addition, a wire through-hole may be formed along the longitudinal direction in the adapter body 2221. In addition, a bending part connection part 2225 may be formed at a longitudinal end of the adapter body 2221.

The spring coupling portion 2222 may be coupled to a longitudinal end of the coil spring 223. For example, the spring coupling portion 2222 may be in the form of a rib protruding along a circumferential direction from an outer circumferential surface of the adapter body 2221. Through this, the end of the coil spring 223 may be caught and supported by the spring coupling portion 2222.

Accordingly, the coil spring 223 may be disposed between the spring coupling portions 2222 formed on the pair of adapters 222, and a restoring force of the coil spring 223 may be applied to the pair of adapters 222.

The wire receiving groove 2223 may be formed along a longitudinal direction on an inner circumferential surface or the outer circumferential surface of the adapter body 2221. For example, four wire receiving grooves 2223 may be formed along the longitudinal direction on the inner circumferential surface of the adapter body 2221.

At least a portion of the bending wire 230 may be accommodated in the wire receiving groove 2223. The bending wire 230 may linearly reciprocate along the longitudinal direction within the wire receiving groove 2223 according to an operation of the operating knob 120.

Although not shown, a wire through-hole may be formed along the longitudinal direction of the adapter body 2221. In this case, at least a portion of the inner circumferential surface of the adapter body 2221 may protrude at a position where the wire receiving groove 2223 is formed, and the wire through-hole may be formed in the protruding portion. That is, the wire through-hole may be disposed at a position connected to the wire receiving groove 2223.

At this time, the bending wire 230 may pass through the wire through-hole.

Meanwhile, the bending part connection portion 2225 may be formed to be coupled to the bending part 221 so as to be relatively rotatable. That is, the bending part connection portion 2225 may be formed with a corresponding convex or concave structure so as to be couplable with the concave portion 2211b of the first bending member 2211 or the convex portion 2212b of the second bending member 2212.

Meanwhile, the coil spring 223 may be disposed to surround an outer circumferential surface of the bending part 221. That is, the coil spring 223 may be disposed in a form of surrounding an outside of the outer circumferential surfaces of the first bending member 2211 and the second bending member 2212 in a state where the first bending member 2211 and the second bending member 2212 are coupled.

On the other hand, both longitudinal ends of the coil spring 223 may be coupled to the adapter 222. The coil spring 223 may be elastically supported by the spring coupling portions 2222 of the pair of adapters 222. Accordingly, when the bending part 221 is bent according to an operation of the operating knob 120, the coil spring 223 may apply an elastic force to restore it to an original position.

Meanwhile, an insertion tube 224 may be disposed at the other side in the longitudinal direction of the insertion tube 220. The insertion tube 224 is formed in a bendable tube shape, and the bending wire 230, the channel 240, and a terminal may pass therethrough. Although not shown, a coil pipe may be provided in the insertion tube 224 to provide an elastic force to the insertion tube 224.

Meanwhile, the bending wire 230 may receive an operating force of the operating knob 120 through the second coupling part 250, and may perform a bending operation of the insertion tube 220 through linear reciprocating movement.

One longitudinal end of the bending wire 230 may be coupled to the adapter 222 or the illumination and imaging unit 210, and the other longitudinal end thereof may be coupled to the second wire frame 256. At this time, the bending wire 230 may pass through the wire through-hole of the adapter 222, the wire through-hole 2211c of the first bending member 2211, and the wire through-hole 2212c of the second bending member 2212.

Accordingly, on a side where the bending wire 230 is pulled, the first bending member 2211 and the second bending member 2212 may be rotated relative to each other such that a gap between the first bending member 2211 and the second bending member 2212 is narrowed. On the other hand, on a side where the bending wire 230 is loosened (lengthened), the first bending member 2211 and the second bending member 2212 may be rotated relative to each other such that the gap between the first bending member 2211 and the second bending member 2212 is widened. Accordingly, the first bending member 2211 and the second bending member 2212 may form a bent shape. In this case, a bending angle between the first bending member 2211 and the second bending member 2212 may be limited according to an angle of the wire guide groove 2212d.

Meanwhile, referring to FIGS. 4 to 13, the second coupling part 250 is detachably coupled to the first coupling part 150 of the operating unit 100. The second coupling part 250 includes a second coupling part body 251, a second coupling plate 255, and a second wire frame 256.

One longitudinal side of the second coupling part body 251 is coupled to the insertion tube 220, and the other longitudinal side thereof may be coupled to the first coupling part 150. In this case, the one longitudinal side of the second coupling part body 251 may be coupled to the other longitudinal side of the insertion tube 220, and the other longitudinal side of the second coupling part body 251 may be provided to be detachably couplable to the first coupling part body 151.

The second coupling part body 251 may be formed in a shape in which a diameter narrows toward one longitudinal end (distal end). As a result, the one longitudinal end of the second coupling part body 251 may be formed in a shape couplable to the insertion tube 224 of the insertion tube 220 to be connected thereto.

The other longitudinal side of the second coupling part body 251 may be provided to engage and couple with the first coupling part 150. For example, the other longitudinal side of the second coupling part body 251 may be in the form of a plate having three surfaces extending in a direction toward the operating unit 100. As another example, the other longitudinal side of the second coupling part body 251 may be in the form of a pair of plates extending in the direction toward the operating unit 100. Accordingly, the second coupling part body 251 may be coupled to the first coupling part body 151 of the first coupling part 150.

As a result, the second coupling part body 251 and the first coupling part body 151 may be fitted into each other. That is, the second coupling part body 251 is coupled to the first coupling part body 151 to accommodate the first coupling plate 155, the first wire frame 156, the second coupling plate 255, and the second wire frame 256 therein.

The second coupling plate 255 may be disposed between a pair of surfaces facing each other among the second coupling part body 251. The second coupling plate 255 is disposed inside the second coupling part body 251 and may be disposed at a position facing the second coupling part body 251.

In addition, the second coupling plate 255 may be disposed at a position facing the first coupling plate 155. In this case, when the first coupling plate 155 is moved according to the movement of the rack gear 154, it may be coupled to the second coupling plate 255.

For example, the second coupling plate 255 may be formed in a square plate shape having a predetermined thickness. In this case, a fixing portion 255a for fixing the coupling with the first coupling plate 155 may be formed on the second coupling plate 255. For example, the fixing portion 255a may be formed in a protrusion shape to be coupled with a hole-shaped fixing portion 155a formed on the first coupling plate 155.

Meanwhile, the second wire frame 256 may be disposed on the second coupling plate 255. The second wire frame 256 may be coupled to the second coupling plate 255 so as to be linearly reciprocable. For example, a guide groove for guiding the linear movement of the second wire frame 256 may be formed in the second coupling plate 255, and the second wire frame 256 may be disposed in the guide groove.

Meanwhile, the bending wire 230 is coupled to the second wire frame 256. For example, the bending wire 230 is coupled to one side in a longitudinal direction of the second wire frame 256, and may be provided to be linearly reciprocable along the longitudinal direction.

Meanwhile, the first wire frame 156 may be coupled to the second wire frame 256. For example, a frame coupling portion 256b may be formed at the other side in a longitudinal direction of the second wire frame 256. The frame coupling portion 256b may be in a form where a plurality of ribs and grooves are alternately formed. The frame coupling portion 256b may be formed in a shape corresponding to the frame coupling portion 156b of the first wire frame 156. That is, the first wire frame 156 and the second wire frame 256 may be coupled in an engaged state with each other.

An assembly in which the first wire frame 156 and the second wire frame 256 are coupled may be disposed inside where the first coupling plate 155 and the second coupling plate 255 are coupled. At this time, the first wire frame 156 is accommodated in a guide groove formed in the first coupling plate 155, and the second wire frame 256 may be accommodated in a guide groove formed in the second coupling plate 255. Therefore, when the first coupling plate 155 and the second coupling plate 255 are coupled, they can surround the assembly of the first wire frame 156 and the second wire frame 256. That is, the assembly of the first wire frame 156 and the second wire frame 256 can reciprocate along a longitudinal direction (front-rear direction), but four sides (up, down, left, right) thereof may be constrained by the first coupling plate 155 and the second coupling plate 255.

Through this, the operating wire 130 coupled to the first wire frame 156 and the bending wire 230 coupled to the second wire frame 256 can move in linkage with each other, and the insertion tube 220 can be bent according to an operation of the operating knob 120.

While the present invention has been described in detail through specific embodiments, these are for describing the present invention in detail, and the present invention is not limited thereto. It is apparent that modifications or improvements can be made by those of ordinary skill in the art within the technical spirit of the present invention.

All simple modifications or changes of the present invention fall within the scope of the present invention, and the specific scope of protection of the present invention will be made clear by the appended claims.

## Claims

1. An endoscope comprising:
an insertion unit including an illumination and imaging unit and an insertion tube; and
an operating unit provided with an operating knob and configured to perform a bending operation of the insertion tube according to an operation of the operating knob,
**characterized in that** the illumination and imaging unit includes:
an illumination and imaging unit body;
an illumination module configured to illuminate light;
a heat sink in contact with the illumination module; and
a heat dissipation chassis provided to surround an outer circumferential surface of the illumination and imaging unit body and disposed to contact the heat sink to dissipate heat of the illumination module.

2. The endoscope of claim 1, wherein the heat sink includes:
a heat sink body; and
an illumination contact portion extending from one end in a longitudinal direction of the heat sink body and contacting the illumination module.

3. The endoscope of claim 2, wherein the illumination and imaging unit further includes an imaging module configured to image an image, and
an imaging module receiving hole for accommodating the imaging module so as to pass therethrough is formed in the heat sink body.

4. The endoscope of claim 3, wherein the heat sink further includes a heat transfer portion extending from the heat sink body in a width direction and contacting the heat dissipation chassis.

5. The endoscope of claim 1, wherein the illumination module includes an illumination lens configured to illuminate light, and
wherein a plurality of the illumination lenses are disposed.

6. The endoscope of claim 5, wherein the illumination and imaging unit further includes an imaging module configured to image an image, and
wherein the plurality of the illumination lenses are symmetrically disposed with respect to the imaging module.

7. The endoscope of claim 5, wherein the plurality of the illumination lenses have different Correlated Color Temperatures (CCT).

8. The endoscope of claim 1, wherein the illumination and imaging unit further includes a heat shield plate configured to block heat of the illumination module.

9. The endoscope of claim 8, wherein the heat shield plate includes:
a shield plate body formed in a semi-circular shape; and
a pair of illumination cover portions symmetrically disposed on the shield plate body and disposed to face the illumination module.

10. The endoscope of claim 9, wherein the illumination and imaging unit further includes an imaging module configured to image an image, and
wherein the heat shield plate further includes a camera cover portion disposed on an axis of symmetry of the pair of the illumination cover portions and disposed at a position facing the imaging module.

11. The endoscope of claim 1, wherein the illumination and imaging unit further includes a printed circuit board configured to control the illumination module, and
wherein the heat sink is in contact with the printed circuit board.

12. An endoscope comprising:
an insertion unit including an illumination and imaging unit and an insertion tube; and
an operating unit provided with an operating knob and configured to perform a bending operation of the insertion tube according to an operation of the operating knob,
**characterized in that** the illumination and imaging unit includes:
a heat dissipation chassis having a cylindrical shape and configured to dissipate heat;
an illumination module configured to illuminate light; and
a heat sink disposed inside the heat dissipation chassis and in contact with the illumination module,
wherein the heat sink is configured to contact the heat dissipation chassis and transfer heat of the illumination module to the heat dissipation chassis.
